Europäisches Patentamt

European Patent Office

Office européen des brevets

(19)

(11) Publication number: **0 028 881**
**A1**

(12) # EUROPEAN PATENT APPLICATION

(21) Application number: **80303404.0**

(22) Date of filing: **26.09.80**

(51) Int. Cl.³: **C 07 C 21/24**
**C 07 C 17/14**

(30) Priority: **09.11.79 GB 7938820**

(43) Date of publication of application:
**20.05.81 Bulletin 81/20**

(84) Designated Contracting States:
**DE FR GB IT SE**

(71) Applicant: **IMPERIAL CHEMICAL INDUSTRIES LIMITED**
**Imperial Chemical House Millbank**
**London SW1P 3JF(GB)**

(72) Inventor: **Morley, John Oswald**
**9 Winchester Close Bamford**
**Rochdale Lancashire(GB)**

(74) Representative: **Pugsley, Roger Graham et al,**
**IMPERIAL CHEMICAL INDUSTRIES LIMITED Legal**
**Department: Patents Thames House North Millbank**
**London SW1P 4QG(GB)**

(54) Preparation of alpha-chloro-ortho-xylene.

(57) A process for the preparation of α-chloro-ortho-xylene by the reaction of chlorine with ortho-xylene in the presence of a phosphorus halide catalyst and a free radical initiator.

EP 0 028 881 A1

**0028881**

This invention relates to a process for the preparation of α-chloro-ortho-xylene.

It has been reported (Olah et al, J.Org.Chem., 39, pp.3472-8, 1974) that the ratio of side chain to ring chlorination in ortho-xylene and a number of other arylalkanes, using $PCl_5$ or $PCl_3$ and chlorine in the dark, is high, with the implication that the phosphorus chlorides are effective catalysts for side chain chlorination in the absence of light or free radicals. Although the conclusions of Olah have been criticised by Messina et al (J.Org.Chem., 44, 2270-4, 1979) who worked with ethylbenzene, the present inventors have repeated the work of Olah and find that side-chain selectivity is indeed exhibited, in the dark and in the absence of free radical initiators, up to conversion levels of about 15% but that, above this level, ring substitution predominates with the result that Olah's conditions are not suitable for the commercial production of α-chloro-ortho-xylene.

According to the present invention there is provided a process for the preparation of α-chloro-ortho-xylene which comprises reacting ortho-xylene with chlorine in the presence of a phosphorus halide and a free radical initiator at from 10°C to 80°C.

The ortho-xylene may be used neat or dissolved in an inert solvent, such as carbon tetrachloride or chlorobenzene, and the chlorine is preferably added in the gaseous state at atmospheric or higher pressures.

The phosphorus halide is preferably phosphorus trichloride or phosphorus pentachloride and especially the latter. The free radical initiator preferably has a half life, at 50°C, of at least 20 hours and as suitable initiators there may be mentioned azo compounds such as azobisisobutyronitrile and peroxides such as dodecanoyl peroxide and benzoyl peroxide.    Although the rate of reaction is to some extent dependent on the concentrations of the phosphorus halide and the free radical initiator, these are only required in catalytic proportions, preferred quantities being from 0.01% to 10%, and especially preferred quantities being from 0.1% to 2.5%, by weight, based on the weight of ortho-xylene.    If necessary, further additions of the initiator may be made during the reaction period in order to maintain this within the desired range.

Although the reaction may be performed at a temperature from 10°C to 80°C it is preferably performed at a temperature from 35°C to 55°C and more preferably from 40°C to 50°C because (with the preferred free radical initiators) the rate of reaction is rather slow at the lower temperatures and the increased rate of dichlorination reduces the yield of the mono—chlorinated product at the higher temperatures.    The rate of reaction may also be varied by adjustment of the rate of addition of chlorine or by a combination of this and the aforementioned rate determining factors.

For commercial efficiency it is preferred to continue the process until the percentage conversion of ortho-xylene into chlorinated products is in the range 20% to 80% and more preferably in the range 30% to 70%. At higher percentage conversions the formation of higher chlorinated xylenes becomes prevalent and efficiency falls off.

The process may be carried out in a batchwise manner but is conveniently performed in a continuous manner and particularly by means of a loop reactor in which the heat of reaction is to some extent dissipated by the recirculating reaction medium.    The desired product may be conveniently separated from unchanged reactants and unwanted by-products by one or more distillation operations.

The process is further illustrated by the following Examples in which the parts and percentages are by weight, unless otherwise indicated.

Example 1

134 Parts of dry chlorine gas are passed into a 1 litre glass reactor containing a well stirred mixture of 500 parts of dry o-xylene, 2.5 parts of phosphorus pentachloride and 2.5 parts of azobisisobutyronitrile in the dark during $1\frac{1}{2}$ hours, and the temperature maintained at 45°C by external cooling. After reaction the solution is degassed with nitrogen and distilled through a Vigreux column initially at 20 mm (mercury) pressure to give 333.5 parts of unchanged o-xylene b.p. 34-36°C (66.7% recovery) and then at 2 mm pressure to give 184 parts of α-chloro-o-xylene, b.p. 48-52°C (84% theory yield based on o-xylene consumed). The 25 parts of residue remaining after distillation contain mainly αα'-dichloro-o-xylene.

Example 2

The procedure of Example 1 is repeated except that the quantity of chlorine is increased to 240 parts and that the 2.5 parts of azobisisobutyronitrile is replaced by 6.2 parts of dodecanoyl peroxide. The reaction mixture is worked up as before to give 202 parts of unchanged o-xylene, b.p. 34-36°C/20 mm Hg (40.4% recovery) and 317 parts of α-chloro-o-xylene, b.p. 48-50°C/2 mm Hg (80% theory yield based on o-xylene consumed).

Example 3

The procedure of Example 1 is repeated except that 550 parts of chlorine are passed into the reaction mixture during $2\frac{1}{2}$ hours at 45°C, a second 2.5 part aliquot of azobisisobutyronitrile is added after 45 minutes' reaction and a third 2.5 part aliquot after $1\frac{1}{2}$ hours. After $2\frac{1}{2}$ hours' reaction almost all of the o-xylene charged has been consumed and the mixture is distilled to give 296 parts of α-chloro-o-xylene, b.p. 48-52°C/2 mm Hg (45% theory). After distillation the remaining 415 parts of residue consists almost entirely of αα'-dichloro-o-xylene.

Example 4

The procedure of Example 1 is repeated except that the quantity of chlorine is increased to 300 parts, the quantity of ortho-xylene is increased to 1000 parts, the quantity of phosphorus pentachloride and azobisbutyronitrile are both increased to 5 parts and the temperature is maintained at $43 \pm 2°C$ throughout the 25 minute reaction period.

After distillation under the conditions set out in Example 1 the xylene recovered amounts to 615 parts (61.5% recovery) and the yield of α-chloro-ortho-xylene is 469 parts (91.8% theory based on xylene consumption). The distillation residue amounts to 47 parts.

Example 5

The procedure of Example 4 is repeated except that the amount of chlorine is increased to 370 parts and this is added over a period of 40 minutes. After distillation under the conditions set out in Example 1 the xylene recovered amounts to 503 parts (50.3% recovery) and the yield of α-chloro-ortho-xylene is 612.6 parts (93% theory based on xylene consumption).

Example 6

The procedure of Example 4 is repeated except that the amount of chlorine is increased to 330 parts and this is added over a period of 35 minutes. After the initial distillation according to Example 1, i.e. at 20 mm Hg, the xylene recovered amounts to 515 parts (51.5% recovery). The residue of 645 parts contains 605 parts of α-chloro-ortho-xylene (yield 95.4% theory based on xylene consumption). This product may be converted directly into ortho-benzyl-toluene, without separation from the small quantity of higher chlorinated products, according to the process described in UK Patent Application No.2009224A.

Example 7

The procedure of Example 1 is repeated except that 1143 parts of chlorine are passed into a 5 litre glass reactor containing 4000 parts of xylene, 30 parts of $PCl_5$ and 30 parts of dodecanoyl peroxide, the latter in place of the 2.5 parts of azobisbutyronitrile, over a period of 90 minutes while the temperature is maintained at $43 \pm 2°C$. After distillation under the conditions set out in Example 1 the xylene recovered amounts to 2560 parts (64% recovery) and the yield of α-chloro-ortho-xylene is 1622 parts (85% theory based on xylene consumption). The chlorine efficiency, i.e. ratio of chlorine consumed to chlorine added, is 90.8%.

Example 8

The procedure of Example 7 is repeated except that the 30 parts of dodecanoyl peroxide are replaced by 30 parts of azobisisobutyronitrile and that 1328 parts of chlorine are added over 105 minutes. After distillation the xylene recovered amounts to 2234 parts (55.9% recovery) and the yield of α-chloro-ortho-xylene is 2124 parts (90.7% based on xylene consumption).   The chlorine efficiency is 94.8%.

**0028881**

Dd.31040/EP

## CLAIMS

1.      A process for the preparation of α-chloro-ortho xylene which comprises reacting ortho-xylene with chlorine in the presence of a phosphorus halide and a free radical initiator at from 10°C to 80°C.

2.      A process according to Claim 1 wherein the free radical initiator has a half life of at least 20 hours at 50°C.

3.      A process according to Claim 2 wherein the free radical initiator is selected from azobisisobutyronitrile, dodecanoyl peroxide and benzoyl peroxide.

4.      A process according to any one of Claims 1 to 3 wherein the proportions of the phosphorus halide and the free radical initiator are from 0.01% to 10%, by weight, based on the weight of the ortho-xylene.

5.      A process according to any one of Claims 1 to 4 performed at a temperature from 35°C to 55°C.

6.      A process according to any one of Claims 1 to 5 performed at a temperature from 40°C to 50°C.

7.      A process according to any one of Claims 1 to 6 wherein the phosphorus halide is phosphorus pentachloride.

8.      A process according to any one of Claims 1 to 7 wherein the percentage conversion of ortho-xylene into chlorinated products is in the range 20% to 80%.

RGP/BH
23.9.80.

# EUROPEAN SEARCH REPORT

**European Patent Office**

EP 80303404.0

| Category | Citation of document with indication, where appropriate, of relevant passages | Relevant to claim |
|---|---|---|
| | GB - A - 583 634 (E.I. DU PONT)<br>+ Claims 1,4,7,9 +<br>--<br>US - A - 2 817 632 (ROWLAND H. MAYOR)<br>+ Claims 1,4,5 +<br>---- | 1 |

**DOCUMENTS CONSIDERED TO BE RELEVANT**

**CLASSIFICATION OF THE APPLICATION (Int. Cl. 3)**

C 07 C 21/24
C 07 C 17/14

**TECHNICAL FIELDS SEARCHED (Int.Cl. 3)**

C 07 C 17/00
C 07 C 21/00

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

| X | The present search report has been drawn up for all claims |
|---|---|

| Place of search | Date of completion of the search | Examiner |
|---|---|---|
| VIENNA | 08-01-1981 | REIF |

EPO Form 1503.1  06.78